# EUROPEAN PATENT SPECIFICATION

(11) **EP 4 321 863 B1**
(45) Date of publication and mention of the grant of the patent: **25.02.2026**
(21) Application number: 22189756.4
(22) Date of filing: 10.08.2022
(51) Int. Cl.: G01N 1/08, G01N 21/65, G01N 21/78, G01N 33/24, G01N 21/71

(54) **METHOD AND DEVICE FOR OBTAINING GEOLOGICAL INFORMATION OF A SOIL SAMPLE AND DEVICE THEREFORE**
VORRICHTUNG UND VERFAHREN ZUR GEWINNUNG GEOLOGISCHER INFORMATIONEN EINER BODENPROBE UND VORRICHTUNG DAFÜR
PROCÉDÉ ET DISPOSITIF D'OBTENTION D'INFORMATIONS GÉOLOGIQUES D'UN ÉCHANTILLON DE SOL ET DISPOSITIF CORRESPONDANT

(43) Date of publication of application: 14.02.2024
(73) Proprietor: Royal Eijkelkamp B.V., 6987 EN Giesbeek (NL)
(72) Inventor: Lichtenberg, Marcus Hermanus, 7206 EV Zutphen (NL); Eijkelkamp, Hugo Jaap, 6987 EM Giesbeek (NL); Klein Lankhorst, Bob, Amsterdam (NL)
(74) Representative: 't Jong, Bastiaan Jacob

(56) References cited:
- CN-A- 106 290 309
- CN-A- 107 966 318
- CN-A- 109 187 082
- CN-A- 114 609 371
- CN-B- 109 827 946
- CN-U- 211 505 501
- US-A1- 2006 139 037
- US-B1- 7 692 789

## Description

The invention relates to a method and a device for obtaining geological information of a soil sample. US 7692789, describes the insertion of a soil sampling tube having a C-shaped cross-section, such that a cutaway portion is provided. After insertion of the soil sampling tube to a predefined depth, the soil sampling tube is retracted from the ground taking along a soil sample enclosed by the soil sampling tube.

A example of a soil sampling and analysis device is furthermore disclosed in CN11460371A.

This soil sampling tube together with the soil sample is then transported off site to a laboratory, where the soil sample is analyzed at discrete positions by a laser induced breakdown spectroscope (LIBS) to determine the elements in the soil sample at the different discrete positions.

When the soil sampling tube together with the soil sample is to be transported off site to a laboratory, it is essential to preserve the soil sample as best as possible. For example any moisture has the tendency to evenly distribute through the sample, especially when the pressure of surrounding soil is no longer present. The same applies for the consistency of the soil sample along the length of the sample. Loose layers would typically fall easily apart during transport and layers, which were pressurized by surrounding layers, can easily expand.

Furthermore, if at a specific site a number of soil samples are to be retrieved, then an equal number of soil sampling tubes is required. All of these sampling tubes need to be transported, which will increase costs substantially, apart from the specialized conditioning equipment required to condition each and every soil sample. However, this is not done in practice. Typically, the sample is just removed from the soil sampling tube and as a result the sample is completely disturbed.

It is an object of the invention to reduce or even remove the above mentioned disadvantages.

This object is achieved by the method according to the invention which method comprises the steps of:
- providing a soil sampling tube having a tip portion, a top portion positioned opposite the tip portion and along the length, between the tip portion and the top portion, a cutaway portion extending along a substantial part of the length of the tube;
- inserting the soil sampling tube into the ground to a predefined depth, while registering the insertion force in combination with the insertion depth of the tip portion;
- while retracting the soil sampling tube with a soil sample out of the ground, analyzing the elements in the soil sample at discrete positions starting adjacent to the top portion end and moving towards the tip portion end of the soil sample tube;
- combining the analyzing results per discrete position starting adjacent to the top portion end and moving towards the tip portion end with the registered insertion force for the insertion depth of the tip portion starting from the insertion to the predefined depth.

With the invention the insertion force is registered when the soil sampling tube is inserted into the ground. This already provides data on the consistency of the soil sample, which is to be retracted from the ground. Then by analyzing the elements in the soil sample at discrete positions as soon as the soil sample is retracted from the ground ensures that the soil sample is as undisturbed as possible and that for example moisture had no chance yet to spread through the sample.

Preferably, the soil sampling tube is rotated around the longitudinal axis at the predefined depth in order to break the sample loose from the ground and prevent loss of the sample, when the soil sampling tube is retracted.

Furthermore, with the method according to the invention, the soil sample is analyzed as soon as the sample is retracted from the ground. This allows for the soil sampling tube to be emptied on site and a number of soil samples can be taken in a short period of time.

In a preferred embodiment of the method according to the invention the elements in the soil sample are analyzed using laser induced breakdown spectroscopy (LIBS) and / or Raman spectroscopy. These analyzing methods allow for a quick analysis at a discrete position of the soil sample, such that the soil sampling tube can be retracted relatively quick.

In yet a further preferred embodiment, an outer layer of the soil sample is removed before the soil sample is analyzed. This ensures that any disturbance of the soil sample by retracting the soil from the ground and / or rotating the soil sampling tube to break the sample loose from the ground has no influence on analyzing the elements in the soil sample.

In a further preferred embodiment, the elements are further analyzed by registering the gamma radiation for each discrete position.

The natural gamma radiation in the soil sample provides also an indication for the soil composition. This contributes is determining the geological information of the sample.

In yet a further embodiment, the soil sample is further analyzed by a microwave sensor for measuring the soil moisture.

A further embodiment of the invention further comprises the step of detecting the geographic position, combining the geographic position with the combined analyzing results and registered insertion force, and storing said combined information on a storage device.

Optionally, also the rotational orientation of the cutaway portion relative to the geographic position is registered. This rotational orientation could be used to determine in combination with other soil samples, the direction and thickness of a layer of an element in the ground.

By combining a plurality of soil samples with the corresponding geographic position, it is possible to generate a three dimensional map of the geological structure of the ground, in which the plurality of soil samples was taken.

The invention also relates to a device for obtaining geological information of a soil sample, which device comprises:
- a base with a support surface for positioning the device with the support surface onto a ground surface;
- a soil sampling tube having a tip portion, a top portion positioned opposite the tip portion and along the length, between the tip portion and the top portion, a cutaway portion extending along a substantial part of the length of the tube;
- guide means arranged to the base and along which the soil sampling tube is guided in a direction perpendicular to the support surface;
- analyzing means arranged to the base, which analyzing means have a detecting area directed to the soil sampling tube, wherein the cutaway portion (6) of the soil sampling tube faces the detecting area of the analyzing means; and a controller, which controls the guide means and the analyzing means.

The device can easily be positioned with the base on a spot on the ground. Then the soil sampling tube is inserted into the ground, while the insertion force is measured. At reaching the desired insertion depth the soil sampling tube is retracted from the ground and the soil sample is analyzed with the analyzing means.

In accordance with the invention the cutaway portion of the soil sampling tube faces the detecting area of the analyzing means. Although the tube could be made of a material transparent for the analyzing means, a better accuracy is obtained, when the analyzing means can examine the soil sample without any obstructive layer in between.

In a further embodiment of the device according to the invention, means for removing an outer layer of the sample are arranged between the base and the analyzing means.

In a very preferred embodiment of the device according to the invention the analyzing means comprises at least one of a laser induced breakdown spectroscope (LIBS) and a Raman spectroscope. These spectroscopes allow for a quick determination of the elements in the soil sample for a specific discrete position.

Preferably, the analyzing means further comprise a gamma ray sensor. Natural gamma rays of the soil sample provide further geological information.

Furthermore, the analyzing means further comprise a microwave sensor for measuring the soil moisture.

Still a further embodiment of the device according to the invention further comprises a camera, preferably a UV sensitive camera, arranged to the base and directed to the cutaway portion of the soil sampling tube.

The images of the camera allow for a further visual inspection of the soil sample at a later time, without the need to preserve the soil sample. Furthermore, the images can be synced with the analyzing results.

These and other features of the inventions will be elucidated in conjunction with accompanying drawings.
Figure 1 shows a schematic perspective view of an embodiment of the device according to the invention.
Figures 2A - 2C show an embodiment of the method according to the invention.

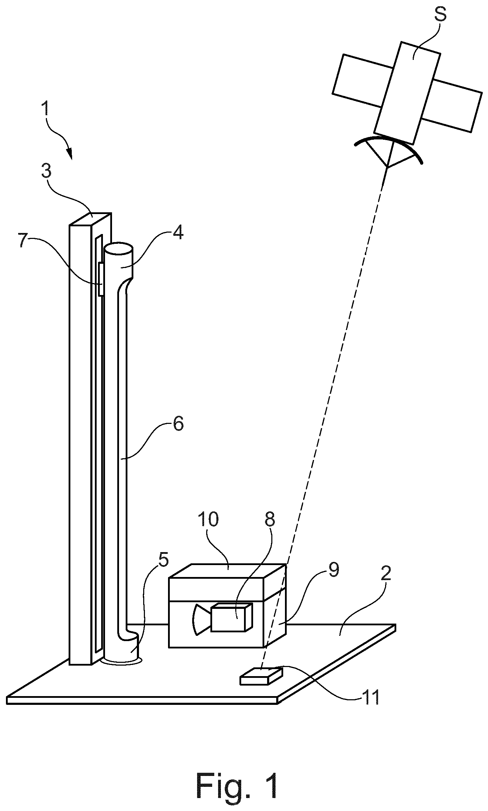

Figure 1 shows an embodiment 1 of a device according to the invention. The device 1 has a base 2 formed by a plate. A guide column 3 is extending vertically from the base 2. A soil sampling tube 4, 5, 6 is arranged parallel and next to the guide column 3 and connected thereto via a connection 7.

The soil sampling tube 4, 5, 6 has a top portion 4, a tip portion 5 and a cutaway portion 6 extending along a substantial part of the length of the tube 4, 5, 6.

Next to the tube 4, 5, 6 a camera 8, a laser induced breakdown spectroscope (LIBS) 9 and a Raman spectroscope 10 are arranged on the base 2. These analyzing devices 8, 9, 10 are each directed to the cutaway portion 6 of the tube 4, 5, 6.

Furthermore, a GPS sensor 11 is arranged on the base 2 to receive a global positioning signal from a satellite S, such that the geographical position of the device 1 can be determined.

The device 1 is controlled by a controller, which controls the guide column 3, the camera 8, the laser induced breakdown spectroscope 9, and the Raman spectroscope 10.

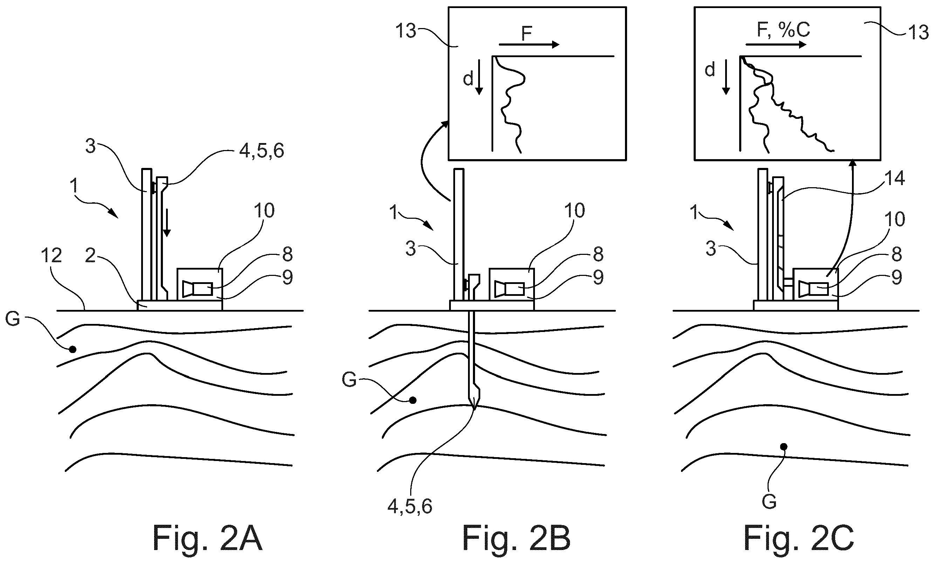

Under control of such a controller, the operation of the device 1 and an embodiment of the method according to the invention is further explained with reference to figures 2A -2C.

In figure 2A, the device 1 is positioned with the base 2 onto a ground surface 12 of the ground G. The controller controls the guide column 3, such that the soil sampling tube 4, 5, 6 is inserted into the ground G. During this movement of the tube 4, 5, 6 into the ground G, the insertion force F is registered, such that a profile 13 relative to the depth d is obtained (see figure 2B)

After the predefined insertion depth is reached, the soil sampling tube 4, 5, 6 is retracted from the ground G by the guide column 3. During this retraction movement, the camera 8, LIBS 9 and Raman spectrometer 10 analyze the soil sample 14 at discrete positions via the cutaway portion 6 of the soil sampling tube 4, 5, 6.

In this example the carbon content of the soil sample 14 is analyzed and combined with the insertion force F into the profile 13.

Using the profile 13 of a number of soil samples performed over an area, it is possible, in combination with the accompanying geographical location determined by the GPS sensor 11, to determine the geological structure of said area.

With the claimed invention, it would also be possible to determine the soil moisture profile over the length of the soil sample. If a soil sample would be transported as is done in the prior art, then the soil moisture profile would already be disturbed by dissipation of the moisture over the length of the soil sample and by evaporation of the moisture.

## Claims

1. Method for obtaining geological information of a soil sample, which method comprises the steps of:
- providing a device according to any of the claims 7-10, said device comprising soil sampling tube (4, 5, 6) having a tip portion (5), a top portion (4) positioned opposite the tip portion (5) and along the length, between the tip portion (5) and the top portion (4), a cutaway portion (6) extending along a substantial part of the length of the tube (4, 5, 6);
- inserting the soil sampling tube (4, 5, 6) into the ground (G) to a predefined depth, while registering the insertion force (F) in combination with the insertion depth of the tip portion (5);
- while retracting the soil sampling tube (4, 5, 6) with a soil sample out of the ground (G), analyzing the elements in the soil sample (14) at discrete positions starting adjacent to the top portion end (4) and moving towards the tip portion end (5) of the soil sample tube (4, 5, 6);
- combining the analyzing results per discrete position starting adjacent to the top portion end (4) and moving towards the tip portion end (5) with the registered insertion force (F) for the insertion depth of the tip portion (5) starting from the insertion to the predefined depth.

2. Method according to claim 1, wherein the elements in the soil sample are analyzed using laser induced breakdown spectroscopy (LIBS) and / or Raman spectroscopy.

3. Method according to claim 2, wherein the elements are further analyzed by registering the gamma radiation for each discrete position.

4. Method according to claim 2 or 3, wherein the soil sample (14) is further analyzed by a microwave sensor for determining the soil moisture.

5. Method according to any of the preceding claims, further comprising the step of removing an outer layer of the soil sample (14) before the soil sample (14) is analyzed.

6. Method according to any of the preceding claims, wherein the method further comprises the step of detecting the geographic position, combining the geographic position with the combined analyzing results and registered insertion force (F), and storing said combined information on a storage device.

7. Device (1) for obtaining geological information of a soil sample (14) , which device (1) comprises:
- a base (2) with a support surface for positioning the device with the support surface onto a ground surface;
- a soil sampling tube (4, 5, 6) having a tip portion (5), a top portion (4) positioned opposite the tip portion (5) and along the length, between the tip portion (5) and the top portion (4), a cutaway portion (6) extending along a substantial part of the length of the tube (4, 5, 6);
- guide means (3) arranged to the base (2) and along which the soil sampling tube (4, 5, 6) is guided in a direction perpendicular to the support surface, which guide means provided with means for registering the insertion force (F) and depth (d);
- analyzing means (8, 9, 10) arranged to the base (2), which analyzing means (8, 9, 10) comprising a detecting area directed to the soil sampling tube (4, 5, 6), wherein the cutaway portion (6) of the soil sampling tube (4, 5, 6) faces the detecting area of the analyzing means (8, 9, 10);
- a controller, adapted to control the guide means (3) and the analyzing means (8, 9, 10).

8. Device (1) according to claim 7, wherein the analyzing means (8, 9, 10) comprises at least one of a laser induced breakdown spectroscope (LIBS) (9) and a Raman spectroscope (10).

9. Device (1) according to claim 7, wherein the analyzing means (8, 9, 10) comprises a gamma ray sensor and/or a microwave sensor.

10. Device (1) according to one of the claims 7 - 9, further comprising a camera (8), preferably a UV sensitive camera, arranged to the base (2) and directed to the cutaway portion (6) of the soil sampling tube (4, 5, 6).

11. Device (1) according to any of the claims 7 - 10, further comprising means for removing an outer layer of the sample (14), which removing means are arranged between the base (2) and the analyzing means (8, 9, 10).

## Patentansprüche

1. Verfahren zum Erhalten geologischer Informationen einer Bodenprobe, wobei das Verfahren die folgenden Schritte umfasst:
- Bereitstellen einer Vorrichtung nach einem der Ansprüche 7-10, wobei die Vorrichtung ein Bodenprobenrohr (4, 5, 6) umfasst, das einen Spitzenabschnitt (5), einen dem Spitzenabschnitt (5) gegenüberliegend positionierten oberen Abschnitt (4) und entlang der Länge zwischen dem Spitzenabschnitt (5) und dem oberen Abschnitt (4) einen ausgeschnittenen Abschnitt (6) aufweist, der sich entlang einen wesentlichen Teil der Länge des Rohrs (4, 5, 6) erstreckt;
- Einstecken des Bodenprobenrohrs (4, 5, 6) in den Boden (G) bis zu einer vordefinierten Tiefe, während die Einsteckkraft (F) in Kombination mit der Einstecktiefe des Spitzenabschnitts (5) erfasst wird;
- während das Bodenprobenrohr (4, 5, 6) mit einer Bodenprobe aus dem Boden (G) zurückgezogen wird, Analysieren der Elemente in der Bodenprobe (14) an diskreten Positionen, beginnend neben dem oberen Abschnittsende (4) und fortlaufend in Richtung des Spitzenabschnittsendes (5) des Bodenprobenrohrs (4, 5, 6);
- Kombinieren der Analyseergebnisse pro diskreter Position, beginnend neben dem oberen Abschnittsende (4) und fortlaufend in Richtung des Spitzenabschnittsendes (5), mit der registrierten Einsteckkraft (F) für die Einstecktiefe des Spitzenabschnitts (5), beginnend bei der Einstecktiefe bis zu der vordefinierten Tiefe.

2. Verfahren nach Anspruch 1, wobei die Elemente in der Bodenprobe mittels laserinduzierter Plasmaspektroskopie (LIBS) und / oder Raman-Spektroskopie analysiert werden.

3. Verfahren nach Anspruch 2, wobei die Elemente weiter analysiert werden, indem die Gammastrahlung für jede diskrete Position registriert wird.

4. Verfahren nach Anspruch 2 oder 3, wobei die Bodenprobe (14) weiter mit einem Mikrowellensensor analysiert wird, um die Bodenfeuchtigkeit zu bestimmen.

5. Verfahren nach einem der vorstehenden Ansprüche, das weiter den Schritt eines Entfernens einer äußeren Schicht der Bodenprobe (14) umfasst, bevor die Bodenprobe (14) analysiert wird.

6. Verfahren nach einem der vorstehenden Ansprüche, wobei das Verfahren weiter den Schritt eines Erkennens der geografischen Position, Kombinierens der geografischen Position mit den kombinierten Analyseergebnissen und der registrierten Einsteckkraft (F) und Speicherns der kombinierten Informationen auf einer Speichervorrichtung umfasst.

7. Vorrichtung (1) zum Erhalten geologischer Informationen einer Bodenprobe (14), wobei die Vorrichtung (1) Folgendes umfasst:
- eine Basis (2) mit einer Auflagefläche, um die Vorrichtung mit der Auflagefläche auf einem Untergrund zu positionieren;
- ein Bodenprobenrohr (4, 5, 6), das einen Spitzenabschnitt (5), einen dem Spitzenabschnitt (5) gegenüberliegend positionierten oberen Abschnitt (4) und entlang der Länge zwischen dem Spitzenabschnitt (5) und dem oberen Abschnitt (4) einen ausgeschnittenen Abschnitt (6) aufweist, der sich entlang einen wesentlichen Teil der Länge des Rohrs (4, 5, 6) erstreckt;
- Führungsmittel (3), die an der Basis (2) angeordnet sind und entlang derer das Bodenprobenrohr (4, 5, 6) in einer Richtung senkrecht zur Auflagefläche geführt wird, wobei die Führungsmittel mit Mitteln zum Erfassen der Einsteckkraft (F) und -tiefe (d) bereitgestellt sind;
- Analysemittel (8, 9, 10), die an der Basis (2) angeordnet sind, wobei die Analysemittel (8, 9, 10) einen auf das Bodenprobenrohr (4, 5, 6) gerichteten Erfassungsbereich umfassen, wobei der ausgeschnittene Abschnitt (6) des Bodenprobenrohrs (4, 5, 6) dem Erfassungsbereich der Analysemittel (8, 9, 10) zugewandt ist;
- eine Steuereinheit, die dazu ausgelegt ist, die Führungsmittel (3) und die Analysemittel (8, 9, 10) zu steuern.

8. Vorrichtung (1) nach Anspruch 7, wobei die Analysemittel (8, 9, 10) mindestens eines von einem laserinduzierten Plasmaspektroskop (LIBS) (9) und einem Raman-Spektroskop (10) umfassen.

9. Vorrichtung (1) nach Anspruch 7, wobei die Analysemittel (8, 9, 10) einen Gammastrahlensensor und/oder einen Mikrowellensensor umfassen.

10. Vorrichtung (1) nach einem der Ansprüche 7 - 9, die weiter eine Kamera (8), vorzugsweise eine UV-empfindliche Kamera, umfasst, die an der Basis (2) angeordnet und auf den ausgeschnittenen Abschnitt (6) des Bodenprobenrohrs (4, 5, 6) gerichtet ist.

11. Vorrichtung (1) nach einem der Ansprüche 7 - 10, die weiter Mittel zum Entfernen einer äußeren Schicht der Probe (14) umfasst, wobei die Entfernungsmittel zwischen der Basis (2) und den Analysemitteln (8, 9, 10) angeordnet sind.

## Revendications

1. Procédé d'obtention d'informations géologiques sur un échantillon de terre, lequel procédé comprend les étapes consistant à :
- fournir un dispositif selon l'une quelconque des revendications 7-10, ledit dispositif comprenant un tube d'échantillonnage de terre (4, 5, 6) présentant une portion de pointe (5), une portion supérieure (4) positionnée à l'opposé de la portion de pointe (5) et le long de la longueur, entre la portion de pointe (5) et la portion supérieure (4), une portion découpée (6) s'étendant le long d'une portion substantielle de la longueur du tube (4, 5, 6) ;
- insérer le tube d'échantillonnage de terre (4, 5, 6) dans le sol (G) à une profondeur prédéfinie, tout en enregistrant la force d'insertion (F) en combinaison avec la profondeur d'insertion de la portion de pointe (5) ;
- tout en rétractant le tube d'échantillonnage de terre (4, 5, 6) avec un échantillon de terre hors du sol (G), analyser les éléments dans l'échantillon de terre (14) à des positions discrètes commençant à côté de l'extrémité de portion supérieure (4) et se déplaçant vers l'extrémité de portion de pointe (5) du tube d'échantillonnage de terre (4, 5, 6) ;
- combiner les résultats d'analyse par position discrète commençant à côté de l'extrémité de portion supérieure (4) et se déplaçant vers l'extrémité de portion de pointe (5) avec la force d'insertion enregistrée (F) pour la profondeur d'insertion de la portion de pointe (5) commençant à partir de l'insertion jusqu'à la profondeur prédéfinie.

2. Procédé selon la revendication 1, dans lequel les éléments dans l'échantillon de terre sont analysés en utilisant la spectroscopie par claquage induit par laser (LIBS) et/ou la spectroscopie Raman.

3. Procédé selon la revendication 2, dans lequel les éléments sont en outre analysés en enregistrant le rayonnement gamma pour chaque position discrète.

4. Procédé selon la revendication 2 ou 3, dans lequel l'échantillon de terre (14) est en outre analysé par un capteur à micro-ondes pour déterminer l'humidité de terre.

5. Procédé selon l'une quelconque des revendications précédentes, comprenant en outre l'étape consistant à retirer une couche extérieure de l'échantillon de terre (14) avant que l'échantillon de terre (14) soit analysé.

6. Procédé selon l'une quelconque des revendications précédentes, dans lequel le procédé comprend en outre l'étape consistant à détecter la position géographique, combiner la position géographique avec les résultats d'analyse combinés et la force d'insertion enregistrée (F), et stocker lesdites informations combinées sur un dispositif de stockage.

7. Dispositif (1) pour obtenir des informations géologiques d'un échantillon de terre (14), lequel dispositif (1) comprend :
- une base (2) avec une surface de support pour positionner le dispositif avec la surface de support sur une surface de sol ;
- un tube d'échantillonnage de terre (4, 5, 6) présentant une portion de pointe (5), une portion supérieure (4) positionnée à l'opposé de la portion de pointe (5) et le long de la longueur, entre la portion de pointe (5) et la portion supérieure (4), une portion découpée (6) s'étendant le long d'une portion substantielle de la longueur du tube (4, 5, 6) ;
- des moyens de guidage (3) agencés sur la base (2) et le long desquels le tube d'échantillonnage de terre (4, 5, 6) est guidé dans une direction perpendiculaire à la surface de support, lesquels moyens de guidage sont pourvus de moyens pour enregistrer la force d'insertion (F) et la profondeur (d) ;
- des moyens d'analyse (8, 9, 10) agencés sur la base (2), lesquels moyens d'analyse (8, 9, 10) comprennent une zone de détection dirigée vers le tube d'échantillonnage de terre (4, 5, 6), dans lequel la portion découpée (6) du tube d'échantillonnage de terre (4, 5, 6) fait face à la zone de détection des moyens d'analyse (8,9,10) ;
- un dispositif de commande, adapté pour commander les moyens de guidage (3) et les moyens d'analyse (8, 9, 10).

8. Dispositif (1) selon la revendication 7, dans lequel les moyens d'analyse (8, 9, 10) comprennent au moins un spectroscope par claquage induit par laser (LIBS) (9) et un spectroscope Raman (10).

9. Dispositif (1) selon la revendication 7, dans lequel les moyens d'analyse (8, 9, 10) comprennent un capteur de rayons gamma et/ou un capteur à micro-ondes.

10. Dispositif (1) selon l'une quelconque des revendications 7-9, comprenant en outre une caméra (8), de préférence une caméra sensible aux UV, agencée sur la base (2) et dirigée vers la portion découpée (6) du tube d'échantillonnage de terre (4, 5, 6).

11. Dispositif (1) selon l'une quelconque des revendications 7-10, comprenant en outre des moyens pour retirer une couche extérieure de l'échantillon (14), lesquels moyens de retrait sont agencés entre la base (2) et les moyens d'analyse (8, 9, 10).
